# EUROPÄISCHE PATENTANMELDUNG

(11) **EP 4 039 211 A1**
(43) Veröffentlichungstag der Anmeldung: **10.08.2022**
(21) Anmeldenummer: 22153932.3
(22) Anmeldetag: 28.01.2022
(51) Int. Cl.: A61B 18/14, A61B 18/00

(54) **RESEKTOSKOP, ELEKTRODENINSTRUMENT FÜR EIN RESEKTOSKOP SOWIE FÜHRUNGSELEMENT FÜR EIN ELEKTRODENINSTRUMENT**

(30) Priorität: 05.02.2021 DE 102021102735
(71) Anmelder: Olympus Winter & Ibe GmbH, 22045 Hamburg (DE)
(72) Erfinder: Brockmann, Christian, 21279 Hollenstedt (DE); Knopf, Christoph, 23554 Lübeck (DE); Offt, Andreas, 21465 Reinbek (DE); Miersch, Hannes, 22149 Hamburg (DE)
(74) Vertreter: Hoener, Matthias

(57) **Zusammenfassung**

Chirurgische Hochfrequenzinstrumente wie beispielsweise Resektoskope (10) werden zum Entfernen oder Manipulieren von Körpergewebe verwendet. Als besonders nachteilig hat es sich bei bekannten Instrumenten gezeigt, dass die Anordnung eines Elektrodeninstruments (22) in einem Innenschaft (12) ein Strömungsverhalten einer Spülflüssigkeit beeinflusse kann. Die Erfindung schafft ein Elektrodeninstrument (22) durch welches die Spülflüssigkeit nicht gestört wird. Das wird dadurch erreicht, dass ein Elektrodeninstrument (22) zwei parallel voneinander angeordnete Elektrodenträger (25, 26) aufweist, die durch mindestens ein Führungselement (27, 32) miteinander verbunden sind und sich dieses mindestens eine Führungselement (27, 32) wenigstens abschnittsweise konvex von einerer Längsachse wegerstreckt (38).

## Beschreibung

Die Erfindung betrifft ein Elektrodeninstrument für ein Resektoskop gemäß dem Oberbegriff des Anspruchs 1. Des Weiteren betrifft die Erfindung ein Führungselement gemäß dem Anspruch 11 sowie ein Resektoskop gemäß dem Anspruch 17.

Chirurgische Hochfrequenzinstrumente wie beispielsweise Resektoskope werden zum Entfernen oder Manipulieren von Körpergewebe verwendet. Typische Anwendungen stellen solche in der Urologie dar. Als ein Beispiel hierfür ist die Prostataresektion zu nennen. Bei einem zum Einsatz kommenden Hochfrequenzwerkzeug kann es sich um eine Elektrode bzw. eine HF-Elektrode handeln, die an einem Hochfrequenzgenerator angeschlossen wird, wobei der Generator mit einem Schalter von einem Operateur aktiviert und deaktiviert werden kann. Der hochfrequente Strom führt dazu, dass sich an der Elektrode ein Plasma bildet. Aufgrund der Wechselwirkung des Plasmas mit dem Gewebe eignen sich HF-Elektroden besonders gut zur punktgenauen Manipulation des Gewebes.

Zum Manipulieren des Gewebes kann die Elektrode anwendungsbezogen als Schneidschlinge oder als Knopfelektrode, aber auch als Nadel, Roller, Band, etc., ausgebildet sein, die sich bei angeschalteter Hochfrequenzspannung aufgrund der hohen Temperatur des Plasmas sehr leicht und beinahe ohne Widerstand durch das zu entfernende Körpergewebe führen lässt.

Die Elektrode ist über ein Elektrodeninstrument mit einem Transporteur bzw. einem Arbeitselement des Resektoskopes lösbar verrastet. Während der Behandlung des Körpergewebes wird das Elektrodeninstrument mit der Elektrode entlang einer Längsrichtung des Resektoskopes bewegt. Je nachdem, ob es sich bei dem Resektoskop um ein aktives oder passives Resektoskop handelt, ist der Transporteur bzw. der Schlitten mit einer Druckfeder bzw. einer Zugfeder mit einem eine Griffeinheit aufweisenden Hauptkörper verbunden.

Bekannte Elektrodeninstrumente werden zusammen mit einer Optik, die als Lichtleiter oder Stablinsensystem ausgebildet sein kann, in einem rohrartigen Schaft, insbesondere einem Innenschaft, des Resektoskopes geführt. Dieser Schaft erstreckt sich vom proximalen Ende bis zum distalen Ende des Resektoskopes und wird zur Behandlung, gegebenenfalls zusammen mit einem Außenschaft, in den zu behandelnden Körper geführt. Innerhalb des Innenschaftes ist das Elektrodeninstrument beweglich an die Optik gekoppelt. Das kann beispielsweise über ein Führungsrohr oder ein Führungsblech erfolgen, welches wenigstens teilweise die Optik umschließt.

Als besonders nachteilig hat es sich bei diesem bekannten Systemen gezeigt, dass die Anordnung des Elektrodeninstruments und der Optik in dem Innenschaft das Strömungsverhalten einer Spülflüssigkeit beeinflusst. Beim Manipulieren des Gewebes können sowohl entstehende Gasblasen als auch Blutungen die Sicht des Operateurs eintrüben. Um dem entgegenzuwirken, wird der Bereich vor dem distalen Ende des Resektoskopes durch die Spülflüssigkeit freigespült. Die Flüssigkeit wird dazu durch den Innenschaft des Resektoskopes geleitet und tritt am distalen Ende aus. Dabei ist es für eine klare Sicht wesentlich, dass sich die austretende Spülflüssigkeit möglichst laminar verhält. Eine optimierte Strömung ist insbesondere erreichbar, wenn die Strömung der Spülflüssigkeit innerhalb des Schaftes nicht gestört wird. Eine Störung kann allerdings durch die Anordnung des Elektrodeninstrumentes um die Optik in dem begrenzten Innenraum des Innenschaftes erfolgen. Die durch den Schaft strömende Flüssigkeit trifft dabei auf das Elektrodeninstrument und/oder die Halterungen und verwirbelt. Diese Verwirbelungen der Spülflüssigkeit führt zu einer turbulenten Strömung, welche besonders nachteilig für die Sicht des Operateurs ist.

Ein weiterer Nachteil bekannter Systeme besteht darin, dass das Führungsblech, insbesondere bei der Montage des Elektrodeninstrumentes, mit anderen Komponenten des Instrumentes verkanntet. Da Stirnflächen des Führungsblechs als auch Stirnflächen anderer Komponenten relativ zu einer Längsachse des chirurgischen Instrumentes senkrecht ausgebildet sind, kann es hier zu einem Verhaken kommen, was zu einer fehlerhaften Montage des Elektrodeninstrumentes, einer Fehlfunktion oder sogar einer Beschädigung der Komponenten führen kann.

Der Erfindung liegt die Aufgabe zugrunde, ein Elektrodeninstrument, ein Führungsblech sowie ein Resektoskop zu schaffen, durch welche die oben genannten Probleme gelöst werden.

Eine Lösung dieser Aufgabe wird durch die Merkmale des Anspruchs 1 beschrieben. Demnach ist es vorgesehen, dass ein Elektrodeninstrument mit einer Elektrode zwei parallel voneinander angeordnete sowie beabstandete Abschnitte eines Elektrodenträgers bzw. jeweils eines Elektrodenträgers aufweist, die wenigstens weitestgehend parallel zu einer Längsachse des Elektrodeninstruments ausgerichtet sind. Ein wesentliches Merkmal der Erfindung besteht darin, dass die beiden Abschnitte durch mindestens ein Führungselement miteinander verbunden sind und sich dieses mindestens eine Führungselement wenigstens abschnittsweise konvex von der Längsachse wegerstreckt. Durch diese Bauweise lässt sich das Elektrodeninstrument besonders kompakt fertigen, sodass ein Querschnitt, den das Elektrodeninstrument in dem Resektoskop einnimmt, besonders klein ist. Außerdem wirkt sich ein gleichbleibender Querschnitt besonders vorteilhaft auf die Anwendung des Instrumentes aus. Dadurch wird die Spülflüssigkeit, die durch das Resektoskop strömt lediglich geringfügig oder gar nicht gestört, sodass am distalen Ende sowie im Inneren des Resektoskopes eine laminare Strömung erzeugt wird, mit der sich besonders vorteilhafte Behandlungsbedingungen erzeugen lassen. Außerdem werden die beiden Abschnitte des Elektrodenträgers bzw. das gesamte Elektrodeninstrument durch das mindestens eine Führungselement stabilisiert. Eine derartige Stabilisierung ist insbesondere vorteilhaft, wenn der Querschnitt der Abschnitte bzw. der Elektrodenhüllrohre sehr gering ist und die Länge der genannten Komponente sehr groß. Durch die gegenseitige Fixierung der Abschnitte durch das Führungselement lassen sich auch besonders dünne sowie besonders lange Elektroden einfach sowie punktgenau einsetzen.

Insbesondere kann es die vorliegende Erfindung vorsehen, dass die beiden Abschnitte als Elektrodenhüllrohre ausgebildet sind, die sich parallel zueinander vom distalen Ende bis zum proximalen Ende des Elektrodeninstruments erstrecken oder dass die beiden Abschnitte gabelartig ausgebildet sind und die Richtung des proximalen Endes des Elektrodeninstrumentes zu einem Elektrodenschaft zusammenlaufen. Durch diesen parallelen Verlauf der Hüllrohre lässt sich das Elektrodeninstrument besonders vorteilhaft parallel zu einem Schaft, wie beispielsweise einem Innenschaft, des Resektoskops ausrichten.

Die Erfindung kann es vorsehen, dass das Elektrodeninstrument mit seinem wenigstens einem Führungselement nicht wie bisher auf der Optik innerhalb des Innenschaftes, sondern vielmehr auf dem Innenschaft des Resektoskopes ablegbar ist und entlang der Längsachse beweglich gelagert ist. Durch diese spezielle Ausgestaltung des Elektrodeninstrumentes lassen sich die Abschnitte der Elektrodenträger bzw. die Elektrodenhüllrohre aus dem Innenschaft auf den Innenschaft verlegen. Somit können die Komponenten des Elektrodeninstrumentes nicht mehr die in den Innenschaft strömende Spülflüssigkeit stören. Vielmehr kann die Flüssigkeit nunmehr ungestört das Resektoskop passieren und die vorteilhafte laminare Strömung ausbilden. Darüber hinaus ist es denkbar, dass durch diese Verlagerung des Elektrodeninstruments der Querschnitt des Innenschafts verringert werden kann.

Vorzugsweise ist es vorgesehen, dass an den beiden Elektrodenhüllrohren zwei oder mehrere Führungselemente angeordnet sind. Durch eine Erhöhung der Anzahl der Führungselemente lässt sich die Stabilität und auch die Handhabung des gesamten Elektrodengerätes verbessern.

Ein weiteres vorteilhaftes Ausführungsbeispiel der Erfindung kann es vorsehen, dass das Führungselement senkrecht zur Längsachse einen bogenartigen, vorzugsweise kreissegmentartigen, Querschnitt aufweist. Dieser Querschnitt kann sich über einen Winkelbereich von 30° - 270°, insbesondere 30° - 180°, vorzugsweise 45° - 120°, oder einen Wert, der zwischen diesen Werten liegt, erstrecken. Durch diesen kreissegmentartigen Querschnitt lässt sich das Elektrodeninstrument besonders einfach und zuverlässig auf eine Außenwandung des Innenschaftes ablegen. Durch diese vorteilhafte Ausgestaltung der Führungselemente ist das Elektrodeninstrument besonders leicht in Richtung der Längsachse über den Innenschaft verschiebbar.

Es kann außerdem vorgesehen sein, dass der Querschnitt des Führungselementes mit seiner Form bzw. seinem Radius mit der Form bzw. dem Radius einer Komponente, insbesondere eines Innenschafts oder einer Optik, des Resektoskopes korrespondiert, sodass das Führungselement auf und/oder unter diese Komponente, insbesondere klemmbar, ist. Das mindestens eine Führungselement ist somit derart ausgebildet, dass sich das Elektrodeninstrument auf eine vorgegebene Art und Weise entlang des Innenschaftes verschieben lässt.

Darüber hinaus ist es erfindungsgemäß denkbar, dass aus dem Führungselement und den Elektrodenhüllrohren ein Hinterschnitt gebildet ist für eine rastende Verbindung mit dem Innenschaft bzw. der Optik. Durch eine elastische Verformung des Führungselements kann das Elektrodeninstrument trotzdem über den Innenschaft bzw. die Optik geklipst werden und wird in Richtung des Zusammenfügens gehalten.

Ein weiteres Ausführungsbeispiel kann es vorsehen, dass das Führungselement um oder auf eine Komponente, vorzugsweise einen Innenschaft oder eine Optik, des Resektoskopes fügbar ist, sodass das Elektrodeninstrument relativ zu der Komponente entlang der Längsachse bewegbar ist.

Bevorzugterweise ist es außerdem denkbar, dass das Führungselement an zwei gegenüberliegenden Seiten kreissegmentartige Aufnahmen aufweist, die mit der Form der Abschnitte bzw. der Hüllrohre korrespondieren, wobei diese Aufnahmen von außen oder innen wenigstens teilweise um die Abschnitte bzw. Hüllrohre fassen. Über diese Aufnahmen lassen sich die Führungselemente besonders einfach sowie flexibel an den Abschnitten befestigen. Dabei ist es denkbar, dass die Aufnahmen sowohl von außen als auch von innen her an den Abschnitten befestigt werden. Darüber hinaus ist es ebenfalls denkbar, dass die Führungselemente über die Aufnahmen nicht lösbar, sondern vielmehr fest an den Abschnitten fixiert werden. Dadurch lässt sich verhindern, dass bei einer größeren Krafteinwirkung parallel oder quer zu der Längsachse die Führungselemente verschoben werden und dadurch die Stabilität des Elektrodeninstruments verringert wird.

Ein besonders bevorzugtes Ausführungsbeispiel der vorliegenden Erfindung sieht es vor, dass ein Abstand zwischen zwei gegenüberliegenden Seiten des Führungselementes 3 mm bis 8 mm beträgt. Durch diese Beabstandung der gegenüberliegenden Seiten lässt sich das Elektrodeninstrument auf eine einfache sowie zuverlässige Art und Weise um den Innenschaft fixieren. Das blechartig ausgebildete Führungselement lässt sich so beispielsweise auf den Schaft aufklipsen. Als Material für das Führungselement eignet sich ein Metall oder ein reversibel verformbarer Kunststoff. Derartige Materialien weisen zum einen die entsprechenden Eigenschaften für die flexible Verbindung des Elektrodeninstruments mit dem Innenschaft auf und sind zum anderen besonders gut zu reinigen. Als Form für das Führungselement eignet sich ein konvex geformtes Rechteck bzw. ein Quader. Es ist jedoch auch denkbar, dass die Führungselemente eine andersartige Form aufweisen. Schließlich muss das Führungselement derart ausgebildet sein, dass es das Elektrodeninstrument entlang des Innenschaftes führen kann.

Ein Führungselement zur Lösung der genannten Aufgabe weist die Merkmale gemäß Anspruch 11 auf. Demnach ist ein Führungselement vorgesehen für ein Elektrodeninstrument eines Resektoskopes nach einem der Ansprüche 1-10. Gekennzeichnet wird dieses Führungselement dadurch, dass es einen bogenartigen, vorzugsweise kreissegmentartigen, Querschnitt aufweist, der mit seiner Form bzw. seinem Radius mit der Form bzw. dem Radius einer Komponente, vorzugsweise eines Innenschaftes oder einer Optik, des Resektoskopes korrespondiert, sodass das Führungselement auf und/oder unter diese Komponente setzbar, insbesondere klemmbar, ist.

Ein bevorzugtes Ausführungsbeispiel des Führungselementes kann es vorsehen, dass diesem an zwei gegenüberliegenden Seiten kreissegmentartige, parallel zueinander ausgerichtete Aufnahmen aufweist, die nach außen oder innen geführt sind und wobei ein Abstand zwischen den beiden überliegenden Seiten oder den Aufnahmen des Führungselementes 3 mm bis 8 mm beträgt. Diese Aufnahmen dienen dazu das Führungselement von außen herum oder von innen nach außen an zwei parallelen Komponenten des Elektrodeninstruments zu befestigen. Durch diese Ausgestaltung des Führungselements ist dieses auf eine einfache und flexible Art und Weise mit dem Elektrodeninstruments koppelbar.

Eine weitere mögliche Ausführungsform der Erfindung kann es vorsehen, dass wenigstens eine, insbesondere in ihrem Querschnitt bogenartig oder kreissegmentartig ausgebildete, Stirnseite senkrecht zu einer Längsachse des Führungselementes ausgebildet ist oder dass die mindestens ein Stirnseite eine Ausnehmung aufweist die sich in die Richtung einer gegenüberliegenden Stirnseite des Führungselementes erstreckt. Durch diese Ausnehmung kann verhindert werden, dass bei einer Montage des Führungselementes bzw. des Elektrodeninstruments selbiges an einer Komponente des Elektrodeninstruments bzw. des Resektoskopes verhakt oder verkanntet. Durch diese Ausnehmung wird die Kontaktfläche zwischen einer Innenseite des Führungselementes und der Komponente bzw. dem Innenschaft des Resektoskopes verringert. Durch diese Verringerung der Kontaktfläche verringert sich auch das Risiko des Verkanntens bzw. Verhakens.

Darüber hinaus ist denkbar, dass die vorgenannte Ausnehmung kreisbogenförmig oder viel eckig, insbesondere dreieckartig, ausgebildet ist. Insbesondere die kreisbogenförmige Ausgestaltung der Ausnehmung vereinfacht die Montage des Elektrodeninstruments, da der Bewegungsspielraum durch die runde Ausnehmung erhöht wird. Dabei ist denkbar, dass die Ausnehmung einen ähnlichen oder einen gleichen Krümmungsradius aufweist wie der Innenschaft.

Weiter ist es denkbar, dass eine an der Ausnehmung anliegende Tangente mit der Längsachse des Führungselementes, vorzugsweise über die gesamte Länge der Stirnseite, einen Winkel α von ungleich 90° einschließt. Durch diese Formgestaltung lässt sich das Risiko des Verkanntens besonders effizient reduzieren.

Vorzugsweise kann es erfindungsgemäß außerdem vorgesehen sein, dass das Führungselement symmetrisch ausgebildet ist, wobei sowohl die beiden Seitenkanten als auch die beiden Stirnseiten mit den Ausnehmungen symmetrisch zueinander ausgebildet sind. Durch diese Symmetrie vereinfachen sich zum einen die Montage der Komponenten und zum anderen das Herstellungsverfahren des Führungselementes.

Schließlich wird durch ein Resektoskop gemäß dem Anspruch 17 eine weitere Lösung der eingangs genannten Aufgabe beschrieben. Dieses Resektoskop weist ein Elektrodeninstrument gemäß den Ansprüchen 1-10 auf. Darüber hinaus verfügt das Resektoskop über einen Innenschaft, in dem eine Optik gelagert ist und wobei das Elektrodeninstrument mit mindestens einem Führungselement gemäß mindestens einem der Ansprüche 11 bis 16 auf dem Innenschaft führbar ist.

Weiter ist es vorzugsweise vorgesehen, dass eine Längsachse des Elektrodeninstrumentes oberhalb oder unterhalb einer Längsachse des Innenschaftes angeordnet ist oder dass eine Längsachse des Elektrodeninstrumentes und eine Längsachse des Innenschaftes in einer Ebene liegen. Dabei ist es denkbar, dass das Führungselement oberhalb der Längsachse des Elektrodeninstrumentes angeordnet und nach unten geöffnet ist oder dass das Führungselement unterhalb der Längsachse des Elektrodeninstrumentes angeordnet und nach oben offen ist. Durch diese Anordnung des Elektrodeninstruments ist lassen sich die einzelnen Komponenten des Resektoskopes besonders platzsparend zueinander ausrichten bzw. positionieren und zwar ohne dass dabei die Spülflüssigkeit in ihrem Strömungsverhalten gestört wird.

Ein bevorzugtes Ausführungsbeispiel der Erfindung wird im Folgenden anhand der Zeichnung näher beschrieben. In dieser zeigen:
- Fig. 1: eine schematische Darstellung eines Resektoskopes,
- Fig. 2: eine schematische, perspektivische Darstellung eines Elektrodeninstrumentes,
- Fig. 3: eine Sicht auf das Elektrodeninstrument gemäß Fig. 2,
- Fig. 4: eine Seitenansicht des Elektrodeninstruments gemäß Fig. 2,
- Fig. 5: eine Sicht auf das distale Ende des Elektrodeninstruments gemäß Fig. 2,
- Fig. 6: eine perspektivische Darstellung eines Führungselementes,
- Fig. 7: eine perspektivische Darstellung eines weiteres Ausführungsbeispiels eines Führungselementes,
- Fig. 8: eine Sicht auf das Führungselement gemäß Fig 7,
- Fig. 9: eine Sicht auf das distale Ende des Elektrodeninstruments gemäß Fig. 7, und
- Fig. 10: eine schematische, perspektivische Darstellung eines Elektrodeninstrumentes mit dem Führungselement gemäß Fig 7.

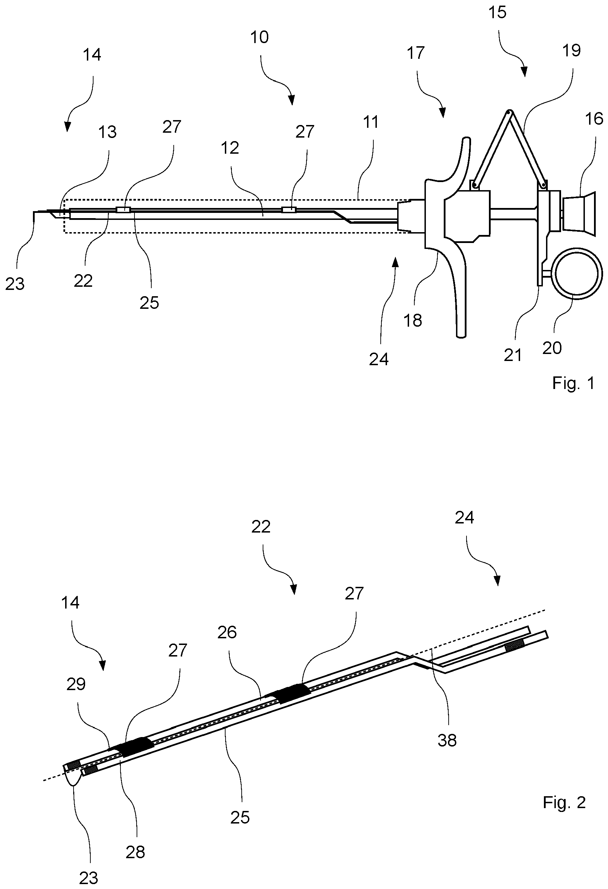

In der Fig. 1 ist ein mögliches Ausführungsbeispiel eines Resektoskopes 10 dargestellt. Bei diesem Resektoskop 10 ist ein äußerer Schaft 11, hier nur durch Strichlinien angedeutet, über einen Innenschaft 12 geschoben. Der Innenschaft 12 dient der Aufnahme bzw. der Führung einer Optik 13, die sich von einem distalen Ende 14 zu einem proximalen Ende 15 des Resektoskopes 10 erstreckt. Am proximalen Ende 15 steht einem Benutzer ein Okular 16 zur Verfügung, um durch die Optik 13 den zu operierenden Bereich vor dem distalen Ende 14 zu beobachten.

Ein wesentlicher Bestandteil des Resektoskopes 10 stellt der Transporteur 17 oder auch Arbeitselement dar. Dieser Transporteur 17 weist unter anderem ein erstes Griffmittel 18 auf und ist über ein Federelement 19 mit einem zweiten Griffmittel 20 sowie einer Optikplatte 21 verbunden.

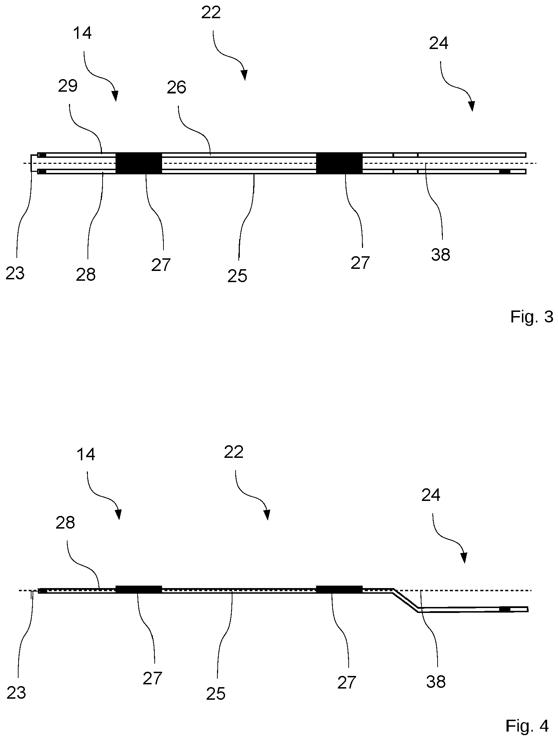

Außerdem erstreckt sich entlang des Innenschafts 12 ein Elektrodeninstrument 22 von dem distalen Ende 14 des Resektoskopes 10 bis zum Transporteur 17. Das in den Fig. 2-4 dargestellte Elektrodeninstrument 22 stellt nur ein mögliches Ausführungsbeispiel dar. Es sei ausdrücklich darauf hingewiesen, dass die hier beschriebene Erfindung nicht auf die hier dargestellte Form beschränkt sein soll. Vielmehr ist es denkbar, dass die beschriebene Erfindung auch im Zusammenhang mit andersartig geformten Elektrodeninstrumenten einsetzbar ist.

Das hier dargestellte Elektrodeninstrument 22 ist mit einem proximalen Ende 24 in dem Transporteur 17 verrastbar. Dadurch lässt sich zum einen das Elektrodeninstrument 22 auf eine einfache Art und Weise von dem Transporteur 17 entkoppeln bzw. an dem Transporteur 17 koppeln und zum anderen mitsamt dem Transporteur 17 entlang der Längsachse des Resektoskopes 10 in distale oder proximale Richtung bewegen.

Am distalen Ende 14 des Elektrodeninstruments 22 weist dieses eine Elektrode 23 auf. Diese Elektrode 23, bzw. Schneidelektrode, ist über einen nicht dargestellten HF-Generator mit elektrischer Energie beaufschlagbar, die dazu dient Gewebe zu manipulieren. Durch die Beaufschlagung der Elektrode mit HF-Spannung bildet sich um die hier als Schneidschlinge dargestellte Elektrode 23 ein Plasma. Durch ein axiales Vor- und Zurückbewegen des Elektrodeninstruments 22 lässt sich das organische Gewebe manipulieren bzw. schneiden. Neben der in den Fig. dargestellten Schneidschlinge sind noch andere Elektrodenformen denkbar.

Für die punktgenaue Manipulation des menschlichen Gewebes ist es von herausragender Bedeutung, dass die Elektrode 23 auf eine sehr präzise Art und Weise handhabbar ist. Diese präzise Handhabung wird insbesondere durch eine Verlängerung des Elektrodeninstrumentes 22 bzw. durch eine Verringerung des Querschnittes des Instrumentes 22 erschwert.

Wie in den Fig. 2 bis 5 ersichtlich, ist die Elektrode 23 mit ihren Enden an zwei Elektrodenträgern 25 und 26 bzw. an zwei Elektrodenhüllrohren mechanisch befestigt bzw. befestigbar. Die Elektrode 23 zusammen mit den Elektrodenträgern 25 und 26 stellen die wesentlichen Bestandteile des Elektrodeninstruments 22 dar. Neben der hier dargestellten parallelen Führung der Elektrodenträger 25 und 26 bzw. der Elektrodenhüllrohre ist es auch denkbar, dass die Elektrodenträger 25 und 26 gabelartig ausgebildet sind und in Richtung des proximalen Endes 15 zu einem Schaft zusammenlaufen.

Neben der mechanischen Verbindung dienen die Elektrodenträger 25 und 26 bzw. die Elektrodenhüllrohre auch zur elektrischen Kontaktierung der Elektrode 23. Es ist vorgesehen, dass sowohl die Elektrodenträger 25 und 26 bzw. die Elektrodenhüllrohre als auch elektrische Leiter innerhalb der Träger 25 und 26 als elektrische Leitungen bzw. Kontakte dienen.

Um die Stabilität und die damit verbundene sichere sowie präzise Handhabung des Elektrodeninstruments 22 zu erhöhen, sieht es die Erfindung vor, wenigstens distale Abschnitte 28, 29 der Elektrodenträger 25, 26 durch ein Führungselement 27 miteinander zu verbinden. Darüber hinaus kann es vorgesehen sein, bzw. ist es denkbar, dass die beiden Elektrodenträger 25, 26 durch mindestens ein weiteres Führungselement 27 miteinander verbunden sind.

Dieses Führungselement 27 dient nicht nur der Stabilisierung des gesamten Elektrodeninstruments 22, sondern auch der Führung entlang des Innenschaftes 12. Erfindungsgemäß wird dabei das mindestens eine Führungselement 27 auf einer Mantelfläche des Innenschaftes 12 abgelegt, wobei das Führungselement 27 den Innenschaft 12 wenigstens teilweise umschließt. Somit ist das Elektrodeninstrument 22 nicht mehr, wie im Stand der Technik, innerhalb des Innenschaftes 12 auf der Optik 13 gelagert, sondern außerhalb des Schaftes 12. Dadurch wird das Innere des Innenschaftes 12 freigegeben für ein störungsfreies Strömen der Spülflüssigkeit. Dies wirkt sich besonders vorteilhaft auf das Strömungsverhalten der Spülflüssigkeit im Bereich vor der Optik 13 außerhalb des Schaftes 11 aus. Die so erzeugte laminare Strömung der Spülflüssigkeit bietet dem Operateur optimale Sichtbedingungen auf den Behandlungsbereich. Gleichermaßen ermöglicht die Verlagerung des Elektrodeninstruments 22 nach außerhalb des Innenschaftes 12 eine Reduzierung des Durchmessers des Innenschaftes 12. Diese Reduzierung des Durchmessers des Innenschaftes 12 wirkt sich positiv auf das gesamte Design des Resektoskops 10 aus, da sich dadurch bspw. auch der Durchmesser eines Außenschaftes des Resektoskops 10 reduzieren lässt. Durch eine Miniaturisierung der Instrumente lässt sich eine besonders körperschonende Behandlung realisieren.

Weiter kann es erfindungsgemäß vorgesehen sein, dass, wie beispielsweise in Fig. 1 dargestellt, dass Elektrodeninstrument 22 über die Führungselemente 27 derart auf den Innenschaft 12 abgelegt wird, dass es oberhalb einer Längsachse des Innenschaftes 12 angeordnet ist. Es hat sich gezeigt, dass diese Anordnung besonders platzsparend ist und gleichermaßen eine präzise Positionierung der Elektrode 23 vor dem distalen Ende des Innenschaftes 12 ermöglicht.

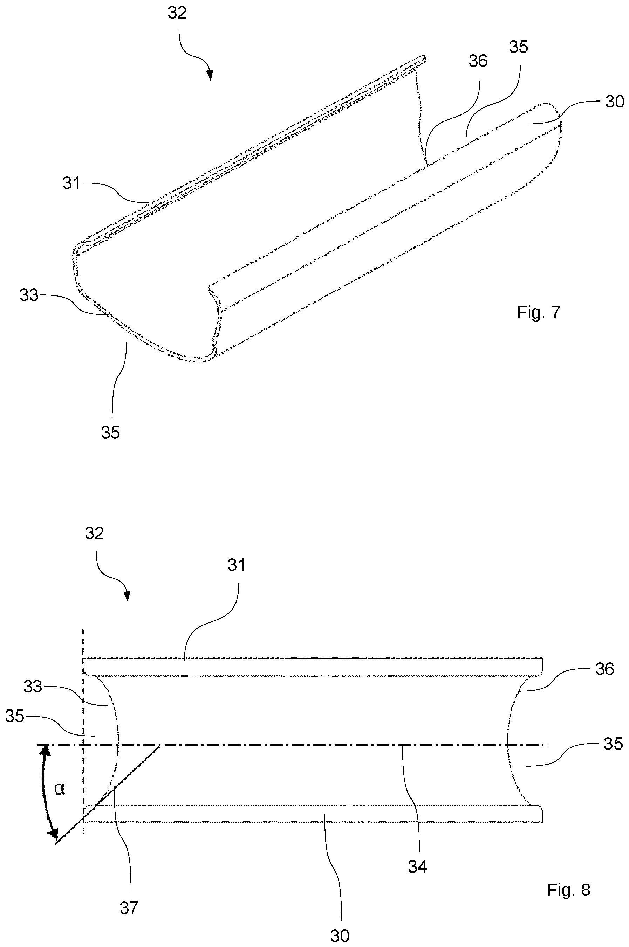

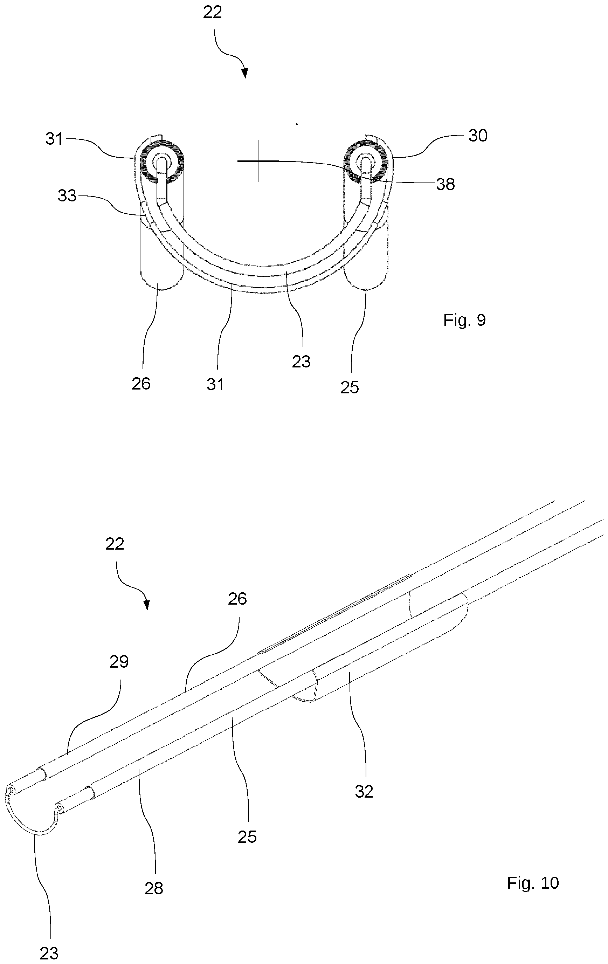

Es ist aber auch denkbar, dass das Führungselement 32 von unten an den Elektrodenträger 25, 26 befestigt werden kann, wobei sich das Elektrodeninstruments 22 sodann unterhalb der Längsachse des Innenschaftes 12 befindet (Fig. 7 - 10). Dadurch ergibt sich der Vorteil, dass die Form des Führungselements 32 einfacher wird, da die kreissegmentartigen Aufnahmen 30, 31 kleiner ausfallen können. Die Aufnahmen 30, 31 sind außerdem in die gleiche Richtung gekrümmt, wie die das Führungselement 32 selbst, womit die Fertigung einfacher wird.

Bevorzugt ist es vorgesehen, dass die freien Enden der Führungselemente 27, 32 in die gleiche Raumrichtung zeigen. So können beim Verschweißen der Führungselemente 27, 32 mit den Elektrodenträgern 25, 26 beide Seiten direkt nacheinander geschweißt werden und zwar ohne die Komponenten dazu drehen zu müssen. Dadurch gestaltet sich das Herstellungsverfahren als besonders effizient.

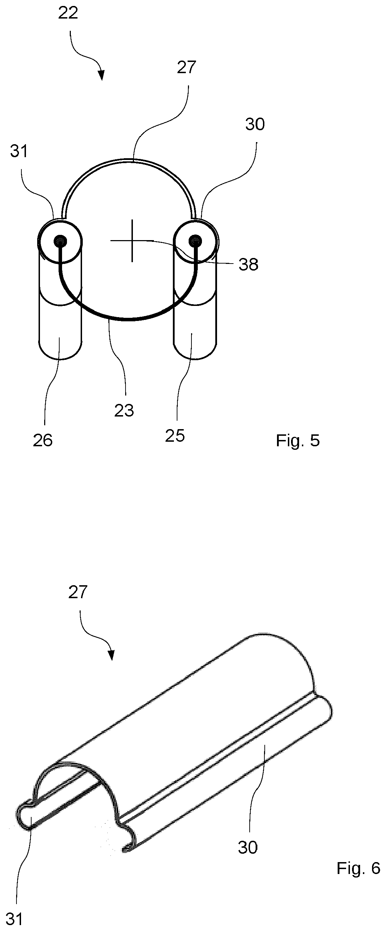

Damit sich das Elektrodeninstrument 22 besonders zuverlässig entlang des Innenschaftes 12 bewegen lässt, ist das Führungselement 27 konvex ausgebildet bzw. bildet in seinem Querschnitt ein Kreissegment (Fig. 5, 6), wobei die konvexe Wölbung von den Elektrodenträger weg zeigt. Der Krümmungsradius des Führungselementes korrespondiert dabei mit der Form bzw. dem Krümmungsradius des Innenschaftes 12. Dadurch fügen sich das Führungselement 27 bzw. die Führungselemente 27 besonders vorteilhaft an die Außenfläche des Innenschaftes 12 an.

Neben der in den Fig. 5 und 6 dargestellten Ausführungsform des Führungselementes 27 ist es auch denkbar, dass der Querschnitt des Führungselementes 27 ein Kreissegment beschreibt, dass größer als 180° ist. Ein derart ausgebildetes Führungselement 27 lässt sich auf den Innenschaft 12 aufspannen bzw. aufklipsen. Ein derart ausgebildetes Führungselement 27 lässt sich auch von unten an den Innenschaft 12 lösbar verbinden (s.o.). Es ist demnach denkbar, dass verschiedene Ausführungsformen von Elektrodeninstrumenten mit den hier beschriebenen Führungselementen 27, 32 von oben oder von unten an dem Innenschaft 12 positioniert werden.

Um das Führungselement 27 zuverlässig mit den Elektrodenträgern 25, 26 zu verbinden, weist dieses an zwei gegenüberliegenden Seiten kreissegmentartige Aufnahmen 30, 31 auf (Fig. 5, 6, 9). Diese Aufnahmen 30, 31 schließen sich wenigstens teilweise, vorzugsweis ganz, um die rohrartigen Elektrodenträger 25, 26. Dabei ist es denkbar, dass die Aufnahmen 30, 31 mit den Elektrodenträgern 25, 26 durch beispielsweise Kleben, Schweißen oder Crimpen verbunden werden.

Das in der Fig. 6 dargestellte Ausführungsbeispiel eines Führungselementes 27 ist derart ausgebildet, dass es sich von oben auf die beiden Elektrodenträger 25, 26 setzen lässt und sich dabei die Aufnahmen 30, 31 um die Elektrodenträger 25, 26 fügen. Durch diese Verbindung des Führungselementes 27 an den Elektrodenträgern 25, 26 wird sichergestellt, dass sich das Elektrodeninstrument 22 auf eine sichere sowie definierte Art und Weise relativ zu dem Innenschaft 12 bewegen lässt.

Ein bevorzugtes Ausführungsbeispiel sieht es vor, dass die Aufnahmen 30, 31 des Führungselementes 27 einen Abstand zwischen 3 und 8 mm zueinander aufweisen. Gleichermaßen es ist denkbar, dass dieser Abstand größer oder kleiner ist. Vorzugsweise ist das Führungselement 27 aus einem Kunststoff oder einem Metall gefertigt, wobei die Wandstärke 0,1 mm bis 1,0 mm, bevorzugt 0,1 mm bis 0,3 mm. Auch eine Wandstärke größer als 2 mm ist denkbar.

Ein weiteres Ausführungsbeispiel für ein erfindungsgemäßes Führungselement 32 ist in den Fig. 7 bis 10 dargestellt. Genau wie bei dem Führungselement 27 weist auch dieses Ausführungsbeispiel längliche, parallel zu einer Längsachse 34 angeordnete Aufnahmen 30, 31 auf. Auch über diese Aufnahmen 30, 31 ist das Führungselement 32 mit dem Elektrodeninstrument 22 verbindbar. Das Führungselement 32 lässt sich sowohl oberhalb als auch unterhalb der Längsachse 38 des Elektrodeninstrumentes 22 über den Innenschaft 12 schieben. Dabei wird genau wie zuvor für das Führungselement 27 beschrieben, auch das Führungselement 32 über die Elektrodenträger 25, 26 geschoben und durch beispielsweise Schweißen fest mit diesen verbunden.

Das in den Fig. 7 und 8 dargestellte Führungselement 32 weist an beiden Stirnseiten 33, 36 jeweils eine Ausnehmung 35 auf. Es ist allerdings auch denkbar, dass nur eine der beiden Stirnseiten 33, 36 eine derartige Ausnehmung 35 aufweist. Wie in der Fig. 8 ersichtlich, sind zur Bildung der Ausnehmung 35 die Stirnseiten 33, 36 bogenartig ausgebildet. Dieser Bogen ist vorzugsweise derart ausgebildet, dass eine an den Stirnseiten 33, 36 anliegende Tangente 37 an nahezu jedem Punkt der Stirnseiten 33, 36 mit der Längsachse 34 einen Winkel α ungleich 90° einschließt. Lediglich an einem gedachten Schnittpunkt zwischen der Längsachse 34 und den Stirnseiten 33, 36 kann der Winkel α gleich 90° betragen. Neben der hier dargestellten bogenartigen Ausgestaltung der Ausnehmung 35 sind auch andere Formen denkbar. So kann es auch vorgesehen sein, dass die Stirnseiten 33, 36 die Form eines Dreiecks aufweisen.

Durch die bogenartig Ausgestaltung der Stirnseiten 33, 36 bzw. durch die Ausnehmungen 35 gestaltet sich die Montage des Führungselementes 32 an dem Elektrodeninstrument 22 bzw. die Montage des Elektroinstrumentes 22 mit dem Führungselement 32 an dem Innenschaft 12 als besonders vorteilhaft. Durch die Ausnehmung 35 wird die Kontaktfläche zwischen dem Führungselement 32 und dem Innenschaft 12 reduziert, sodass die Gefahr eines Vakantens bzw. Verhakens ebenfalls reduziert wird.

**Bezugszeichenliste:**

| | | | |
|---|---|---|---|
| 10 | Resektoskop | 29 | Abschnitt |
| 11 | Schaft | 30 | Aufnahme |
| 12 | Innenschaft | 31 | Aufnahme |
| 13 | Optik | 32 | Führungselement |
| 14 | Distales Ende | 33 | Stirnseite |
| 15 | Proximales Ende | 34 | Längsachse |
| 16 | Okular | 35 | Ausnehmung |
| 17 | Transporteur | 36 | Stirnseite |
| 18 | Erstes Griffmittel | 37 | Tangente |
| 19 | Federelement | 38 | Längsachse des |
| 20 | Zweites Greifmittel | | Elektrodeninstrumentes |
| 21 | Optikplatte | | |
| 22 | Elektrodeninstrument | | |
| 23 | Elektrode | | |
| 24 | Proximales Ende | | |
| 25 | Elektrodenträger | | |
| 26 | Elektrodenträger | | |
| 27 | Führungselement | | |
| 28 | Abschnitt | | |

## Patentansprüche

1. Elektrodeninstrument (22) für ein Resektoskop (10) mit einer Elektrode (23), wobei die Elektrode (23) an zwei distalen Enden zweier rohrartigen sowie parallel voneinander beabstandeten Abschnitten (28, 29) jeweils eines Elektrodenträgers (25, 26) befestigt ist und die Abschnitte (28, 29) wenigstens weitestgehend parallel zu einer Längsachse (38) des Elektrodeninstrumentes (22) ausgerichtet sind, **dadurch gekennzeichnet, dass** an den Elektrodenträgern (25, 26) mindestens ein Führungselement (27, 32) angeordnet ist, welches die beiden Abschnitte (28, 29) miteinander verbindet, wenigstens abschnittsweise konvex ist und sich von der Längsachse (38) wegerstreckt.

2. Elektrodeninstrument (22) nach Anspruch 1, **dadurch gekennzeichnet, dass** die beiden Abschnitte (28, 29) als Elektrodenhüllrohre ausgebildet sind, die sich parallel zueinander vom distalen Ende bis zum proximalen Ende (24) des Elektrodeninstrumentes (22) erstrecken oder dass die beiden Abschnitte (28, 29) gabelartig ausgebildet sind und in Richtung des proximalen Endes (24) des Elektrodeninstrumentes (22) zu einem Elektrodenschaft zusammenlaufen.

3. Elektrodeninstrument (22) nach Anspruch 2, **dadurch gekennzeichnet, dass** an den beiden Elektrodenhüllrohren zwei oder mehr Führungselemente (27, 32) angeordnet sind.

4. Elektrodeninstrument (22) nach einem der vorangegangenen Ansprüche, **dadurch gekennzeichnet, dass** das Führungselement (27, 32) senkrecht zur Längsachse (38) einen bogenartigen, vorzugsweise kreissegmantartigen, Querschnitt aufweist.

5. Elektrodeninstrument (22) nach Anspruch 4, **dadurch gekennzeichnet, dass** der Querschnitt mit seiner Form bzw. seinem Radius mit der Form bzw. dem Radius einer Komponente, insbesondere eines Innenschaftes (12) oder einer Optik (13), des Resektoskops (10) korrespondiert, so dass das Führungselement (27, 32) auf und/oder unter diese Komponente setzbar, insbesondere klemmbar, ist.

6. Elektrodeninstrument (22) nach einem der vorangegangenen Ansprüche, **dadurch gekennzeichnet, dass** aus dem Führungselement (27, 32) und den Elektrodenhüllrohren ein Hinterschnitt gebildet ist für eine rastende Verbindung mit dem Innenschaft (12) bzw. der Optik (13).

7. Elektrodeninstrument (22) nach einem der vorangegangenen Ansprüche, **dadurch gekennzeichnet, dass** das Führungselement (27, 32) um oder auf eine Komponente, vorzugsweise einen Innenschaft (12) oder eine Optik (13), des Resektoskopes (10) fügbar ist, so dass das Elektrodeninstrument (22) relativ zu der Komponente entlang der Längsachse (38) bewegbar ist.

8. Elektrodeninstrument (22) nach einem der vorangegangenen Ansprüche, **dadurch gekennzeichnet, dass** das Führungselement (27, 32) an zwei gegenüberliegenden Seiten kreissegmentartige Aufnahmen (30, 31) aufweist, die mit der Form der Abschnitte (28, 29) bzw. der Elektrodenhüllrohre korrespondieren, wobei diese Aufnahmen (30, 31) von außen oder innen wenigstens teilweise um die Abschnitte (28, 29) bzw. Elektrodenhüllrohre fassen.

9. Elektrodeninstrument (22) nach einem der vorangegangenen Ansprüche, **dadurch gekennzeichnet, dass** das Führungselement (27, 32) oberhalb oder unterhalb der Längsachse (38) des Elektrodeninstrumentes (22) an den Abschnitten (28, 29) anordbar ist, wobei das Führungselement (27, 32) oberhalb der Längsachse (38) des Elektrodeninstrumentes (22) angeordnet und nach unten geöffnet ist oder das Führungselement (27, 32) unterhalb der Längsachse (38) des Elektrodeninstrumentes (22) angeordnet und nach oben offen ist.

10. Elektrodeninstrument (22) nach einem der vorangegangenen Ansprüche, **dadurch gekennzeichnet, dass** ein Abstand zwischen zwei gegenüberliegenden Seiten des Führungselementes (27) 3 mm bis 8 mm beträgt.

11. Führungselement (27, 32) für ein Elektrodeninstrument (22) eines Resektoskopes (10) nach einem der vorangegangenen Ansprüche 1 bis 10, **gekennzeichnet durch** einen bogenartigen, vorzugsweise kreissegmentartigen, Querschnitt, der mit seiner Form bzw. seinem Radius mit der Form bzw. dem Radius einer Komponente, vorzugsweise eines Innenschaftes (12) oder einer Optik (13), des Resektoskopes (10) korrespondiert, so dass das Führungselement (27, 32) auf und/oder unter diese Komponente setzbar, insbesondere klemmbar, ist.

12. Führungselement (27, 32) für ein Elektrodeninstrument (22) nach Anspruch 11, **dadurch gekennzeichnet, dass** das Führungselement (27, 32) an zwei gegenüberliegenden Seiten kreissegmentartige, parallel zueinander ausgerichtete Aufnahmen (30, 31) aufweist, die nach außen oder innen geführt sind und wobei ein Abstand zwischen den zwei gegenüberliegenden Seiten oder den Aufnahmen (30, 31) des Führungselementes (27, 32) 3 mm bis 8 mm beträgt.

13. Führungselement (27, 32) für ein Elektrodeninstrument (22) nach Anspruch 11 oder 12, **dadurch gekennzeichnet, dass** wenigstens eine, insbesondere in ihrem Querschnitt bogenartig oder kreissegmentartig ausgebildete, Stirnseite (33, 36) senkrecht zu einer Längsachse (34) des Führungselementes (27, 32) ausgebildet ist oder dass die mindestens eine Stirnseite (33, 36) eine Ausnehmung (35) aufweist, die sich in die Richtung einer gegenüberliegenden Stirnseite (33, 36) erstreckt.

14. Führungselement (27, 32) für ein Elektrodeninstrument (22) nach Anspruch 13, **dadurch gekennzeichnet, dass** die Ausnehmung (35) kreisbogenförmig oder vieleckig, insbesondere dreieckartig, ausgebildet ist.

15. Führungselement (27, 32) für ein Elektrodeninstrument (22) nach Anspruch 13 oder 14, **dadurch gekennzeichnet, dass** eine an der Ausnehmung (35) anliegende Tangente (37) mit der Längsachse (34), vorzugsweise über die gesamte Länge der Stirnseite (33, 36), einen Winkel α von ungleich 90° einschließt.

16. Führungselement (27, 32) für ein Elektrodeninstrument (22) nach einem der Ansprüche 11 bis 15, **dadurch gekennzeichnet, dass** das Führungselement (27, 32) symmetrisch ausgebildet ist, wobei sowohl die beiden Seitenkanten als auch die beiden Stirnseiten (33, 37) mit den Ausnehmungen (35) symmetrisch zueinander ausgebildet sind.

17. Resektoskop (10) mit einem Elektrodeninstrument (22) nach einem der vorangegangenen Ansprüche 1 bis 10 und einem Innenschaft (12), in dem eine Optik (13) gelagert ist, wobei das Elektrodeninstrument (22) mit mindestens einem Führungselement (27, 32) gemäß mindestens einem der Ansprüche 11 bis 16 auf dem Innenschaft (12) führbar ist.

18. Resektoskop (10) nach Anspruch 17, **dadurch gekennzeichnet, dass** eine Längsachse (38) des Elektrodeninstrumentes (22) oberhalb oder unterhalb einer Längsachse des Innenschafts (12) liegt oder dass eine Längsachse (38) des Elektrodeninstrumentes (22) und eine Längsachse des Innenschafts (12) in einer Ebene liegen.
